(19) Europäisches Patentamt
European Patent Office
Office européen des brevets

(11) Veröffentlichungsnummer: **0 096 286**
**A2**

# (12) EUROPÄISCHE PATENTANMELDUNG

(21) Anmeldenummer: **83105108.1**

(22) Anmeldetag: **24.05.83**

(51) Int. Cl.³: **A 61 K 9/24**
A 61 K 31/535

(30) Priorität: **07.06.82 DE 3221425**

(43) Veröffentlichungstag der Anmeldung:
**21.12.83 Patentblatt 83/51**

(84) Benannte Vertragsstaaten:
**AT BE CH DE FR IT LI LU NL SE**

(71) Anmelder: **BOEHRINGER INGELHEIM KG**
**ZA Patente**
**D-6507 Ingelheim am Rhein(DE)**

(72) Erfinder: **Schmidt, Michael, Dr.**
**Hammergasse 88**
**D-6507 Ingelheim/Rhein(DE)**

## (54) Hydrolyseempfindlichen Wirkstoff enthaltende, lagerstabile Tablette.

(57) Lagerstabile Tablette, welche einen hydrolyseempfindlichen Wirkstoff enthält, die aus mehreren Schichten besteht, von denen lediglich eine wirkstoffhaltig ist.

EP 0 096 286 A2

Croydon Printing Company Ltd

Gegenstand der Erfindung ist eine stabile pharmazeutische Zubereitung eines hydrolyseempfindlichen Wirkstoffes in einer festen komprimierten Arzneiform sowie Verfahren zu deren Herstellung. Insbesondere betrifft die Erfindung eine lagerstabile Tablette, welche einen hydrolyseempfindlichen Wirkstoff enthält und Verfahren zu deren Herstellung.

Zur Stabilisierung hydrolyseempfindlicher Substanzen sind in der Literatur viele Verfahren beschrieben, die sich auf die folgenden Grundoperationen zurückführen lassen:

1. Entfernung von Wasser aus der Formulierung
   Hier sind in erster Linie die Sprühtrocknung von Wirkstoff-Hilfsstoff-Lösungen bzw. -Suspensionen oder andere Trocknungsverfahren zu nennen, die am fertigen Produkt oder im Verlaufe der Herstellung zur Anwendung gelangen.

2. Einhüllen der Wirkstoffe
   Als übliche Verfahren wären hier die Mikroverkapselung oder das Aufsprühen von Polymeren auf Wirkstoffe in geeigneten Apparaturen zu nennen.

3. Hydrophobierung der Wirkstoffe
   Bei diesen Verfahren reicht die Palette von einfachen Mischungen der Wirkstoffe mit hydrophoben Hilfsstoffen bis zu Schmelzeinbettungen in wachs- oder fettartigen Substanzen.

4. Einstellung eines optimalen pH-Wertes
   Diese für Lösungen einfache und übliche Methode, die Hydrolysegeschwindigkeit zu minimieren, wird für feste Arzneiformen selten beschrieben. Häufiger wird generell der Zusatz sauer- oder basisch reagierender Hilfsstoffe vorgeschlagen, ohne daß eine direkte pH-Einstellung erfolgt.

Die genannten Verfahren haben verschiedenartige Nachteile, von denen im folgenden einige genannt werden sollen:

Die unter 1. genannten Verfahren haben neben der thermischen Belastung der Wirkstoffe den Nachteil, daß alle sich der Trocknung anschließenden Verfahrensschritte, z.B. die Verpackung, unter feuchtearmen Bedingungen durchgeführt werden müssen, da getrocknete Materialien im allgemeinen in sehr kurzer Zeit wieder Wasser aus der Luft aufnehmen können.

Insgesamt führen diese Verfahren zu einer Verteuerung des Herstellungsprozesses und erfordern einen größeren technischen Aufwand.

Die unter Punkt 2 genannten Verfahren sind ebenfalls kostenintensiv; außerdem ist es häufig erforderlich, mit organischen Lösungsmitteln zu arbeiten, so daß die Anforderungen des Umwelt- und Explosionsschutzes beachtet werden müssen.

Die unter Punkt 3 genannten Verfahren führen häufig zu einer Verschlechterung der Wirkstoff-Freigabe aus der Arzneiform. Der Einfluß auf die biopharmazeutischen Auswirkungen muß von Fall zu Fall untersucht werden und ist normalerweise sehr stark vom Wirkstoff abhängig.

Die unter 4 genannte Einstellung des pH-Wertes läßt sich nur über die Auflösung bzw. Suspendierung der festen Form kontrollieren. In der festen Arzneiform selbst müssen lokale pH-Unterschiede einkalkuliert werden.

Die vorliegende Erfindung macht sich nun die Tatsache zunutze, daß die Hydrolysegeschwindigkeit eines Wirkstoffes

in einer festen Arzneiform häufig auch von der Konzentration des Wirkstoffes abhängt. Besonders deutlich ist diese Abhängigkeit bei einem Sydnonimin-Derivat, dem N-Cyclohexancarbonyl-3-(4-morpholino)-sydnonimin-hydrochlorid der Formel

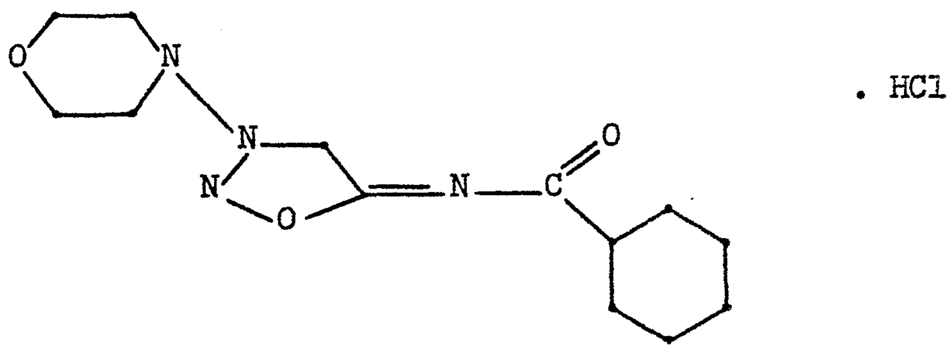

festzustellen. Auf übliche Weise durch Feuchtgranulation erhaltene Tabletten, die dieses Syndonimin-Derivat enthalten, zeigten nach 14 monatiger Lagerung bei 26$^{0}$C in einem feuchtigkeitsdichten Packmittel in Abhängigkeit vom Wirkstoff-Hilfsstoff-Verhältnis die folgenden Abnahmen des Wirkstoff-Gehaltes

| Wirkstoffmenge pro Tablette | Gewichtsverhältnis Wirkstoff : Hilfsstoffe | Gehaltsabnahmen in Gew.-% des Wirkstoffes |
|---|---|---|
| 10 mg | 1 : 8 | - |
| 5 mg | 1 : 16 | 4 % |
| 2,5 mg | 1 : 32 | 20 % |

Es konnte ferner nachgewiesen werden, daß auch bei Tabletten, die durch Direktverpressung ohne Wasserzugabe hergestellt wurden, bei längerer Lagerung eine Wirkstoffzersetzung eintritt.

Um für die niedrigste Dosierung von 2,5 mg auf ein stabiles Wirkstoff-Hilfsstoff-Verhältnis zu gelangen, müßte demnach

der Wirkstoffgehalt auf mindestens 1 zu 8 erhöht und demnach Komprimate mit einem Einzelgewicht von 20 mg hergestellt werden. Die Herstellung entsprechender Tabletten wäre zwar möglich, jedoch wären an die Fließeigenschaften der Rezeptur sehr hohe Forderungen zu stellen, um eine ausreichende Homogenität der Tablettengewichte und eine vertretbare Streuung der Wirkstoff-Gehalte zu erzielen. Die erforderlichen Preßstempel entsprechender Tablettenpressen mit Durchmessern von 3 bis 4 mm sind sehr empfindlich, so daß die resultierenden Tabletten aufgrund der kleinen Abmessungen schlecht zu handhaben sind und sich als problematisch beim Verpackungsvorgang erweisen. Eine Teilbarkeit zur individuelleren Dosierung wäre nicht gegeben.

Ziel der Erfindung ist daher eine lagerstabile Tablette, welche einen hydrolyseempfindlichen Wirkstoff enthält, für deren Herstellung die üblichen Hilfsstoffe eingesetzt werden können und auf kostenintensive Arbeitsschritte wie Sprühtrocknung und dergleichen verzichtet werden kann. Ferner sollen die Vorteile normal dimensionierter Tabletten wie Handhabbarkeit und Teilbarkeit beibehalten bleiben.

Dieses Ziel kann erfindungsgemäß durch eine Tablette erreicht werden, die aus mehreren Schichten besteht, von denen lediglich eine wirkstoffhaltig ist. Bevorzugt wird eine Tablette, die aus mindestens einer wirkstofffreien und einer wirkstoffhaltigen Schicht besteht.

Insbesondere wird erfindungsgemäß eine Tablette vorgeschlagen, die als Wirkstoff N-Cyclohexancarbonyl-3-(4-morpholino)-sydnonimin-hydrochlorid in einer (Verum-) Schicht neben mindestens einer wirkstofffreien (Placebo-) Schicht enthält. Besonders bevorzugt ist eine Tablette mit dem vorgenannten Sydnonimin als Wirkstoff, die ein Gesamtgewicht von mindestens 40 mg hat und den Wirkstoff in einer Konzentration in der Verum-Schicht von mindestens 12,5 Gew.-% enthält.

Die beiliegenden Zeichnungen verdeutlichen die Erfindung: Figur 1 zeigt eine Zweischicht-Tablette, die aus etwa gleichgroßen Anteilen einer Verum-Schicht (1) und einer Placebo-Schicht (2) besteht. In der Figur 2 ist eine Dreischicht-Tablette dargestellt, die außen zwei Placebo-Schichten (2) und in der Mitte eine Verum-Schicht (1) trägt.

Tabletten der in den Figuren 1 und 2 dargestellten Art können nach der üblichen Art der Herstellung von Mehrschicht-Tabletten erhalten werden. So ist z.B. eine Zweischicht-Tablette in der Weise herstellbar, daß zunächst eine bestimmte Menge wirkstofffreies Granulat bzw. wirkstofffreie Pulvermischung in die Matrize eingefüllt und leicht komprimiert wird. Danach wird die entsprechende Menge der wirkstoffhaltigen Rezeptur auf diese Placebo-Schicht gefüllt und die eigentliche Verpressung durchgeführt. Das umgekehrte Vorgehen ist ebenfalls möglich. Zunächst wird die Verum-Rezeptur eingefüllt und vorkomprimiert, anschließend wird mit der Placebo-Rezeptur aufgefüllt und die eigentliche Kompression durchgeführt.

Bei weißem Wirkstoff ist es der Tablette von außen nicht anzusehen, daß es sich hierbei um eine Zweischicht-Tablette handelt. Selbstverständlich ist die Anfärbung einer oder beider Schichten möglich, um das Vorliegen einer Mehrschicht-Tablette kenntlich zu machen.

Für die Herstellung einer Dreischicht-Tablette wird zunächst ein Teil der Placebo-Rezeptur in die Matrize eingefüllt und vorkomprimiert. Danach wird die erforderliche Menge an wirkstoffhaltiger Rezeptur eingefüllt und erneut vorkomprimiert. Zuletzt wird die restliche Menge an

Placebo-Rezeptur auf die beiden Schichten gegeben und die eigentliche Kompression durchgeführt.

Das folgende Beispiel erläutert die Erfindung in nicht beschränkender Weise

Beispiel 1

Tablette mit N-Cyclohexancarbonyl-3-(4-morpholino)-sydnonimin-hydrochlorid als Wirkstoff

| | wirkstoffhaltige Schicht | Placebo-Schicht |
|---|---|---|
| Granulat: | | |
| Wirkstoff | 2,5 mg | - |
| Milchzucker | 6,0 mg | 12,75 mg |
| Mikrokristalline Cellulose | 4,6 mg | 6,90 mg |
| Maisstärke | 5,4 mg | 8,10 mg |
| | 18,5 mg | 27,75 mg |
| Zumischung: | | |
| Maisstärke | 1,3 mg | 1,95 mg |
| Aerosil | 0,1 mg | 0,15 mg |
| Magnesiumstearat | 0,1 mg | 0,15 mg |
| Menge/Schicht | 20,0 mg | 30,00 mg |

Zunächst wird aus Wirkstoff, Milchzucker, Cellulose und Maisstärke durch übliche Feuchtgranulation ein Granulat hergestellt, welches anschließend mit Maisstärke, Aerosil und Magnesiumstearat vermischt wird.

In der gleichen Weise wird die Placebo-Rezeptur hergestellt.

In die Matrize einer geeigneten Tablettenpresse werden 30 mg der Placebo-Rezeptur eingefüllt und leicht vorkomprimiert. Auf diese Schicht werden dann 20 mg der wirkstoffhaltigen Rezeptur gegeben und die Tabletten gepreßt.

Nach 14 Monaten bei 26°C in feuchtigkeitsdichtem Packmittel wurde keine signifikante Abnahme des Wirkstoffgehaltes festgestellt.

PATENTANSPRÜCHE:

1. Lagerstabile Tablette, welche einen hydrolyseempfindlichen Wirkstoff enthält, dadurch gekennzeichnet, daß sie aus mehreren Schichten besteht, von denen lediglich eine wirkstoffhaltig ist.

2. Tablette nach Anspruch 1, dadurch gekennzeichnet, daß sie aus mindestens einer wirkstofffreien und einer wirkstoffhaltigen Schicht besteht.

3. Tablette nach Anspruch 1 und/oder 2, dadurch gekennzeichnet, daß sie als Wirkstoff N-Cyclohexancarbonyl-3-(4-morpholino)-sydnonimin-hydrochlorid in einer (Verum-) Schicht neben mindestens einer wirkstofffreien (Placebo-) Schicht enthält.

4. Tablette nach Anspruch 3, dadurch gekennzeichnet, daß sie ein Gesamtgewicht von mindestens 40 mg hat und den Wirkstoff in einer Konzentration in der Verumschicht von mindestens 12,5 Gew.-% enthält.

5. Verfahren zur Herstellung einer lagerstabilen Tablette nach einem der Ansprüche 1 bis 5, dadurch gekennzeichnet, daß man auf einer Tablettenpresse eine Mehrschicht-Tablette herstellt, bei welcher lediglich eine Schicht wirkstoffhaltig ist.

1/1

0096286

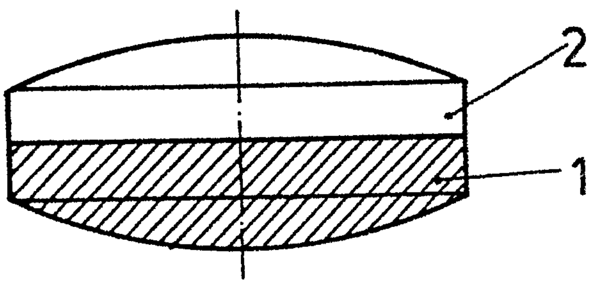


FIG. 1

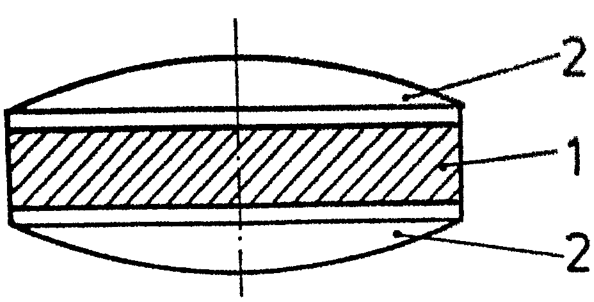


FIG. 2